# EUROPÄISCHE PATENTSCHRIFT

(11) **EP 4 288 727 B1**
(45) Veröffentlichungstag und Bekanntmachung des Hinweises auf die Patenterteilung: **22.01.2025**
(21) Anmeldenummer: 21789603.4
(22) Anmeldetag: 30.09.2021
(51) Int. Cl.: F25D 17/04

(54) **CRYOSAUNA**
CRYOSAUNA
CRYOSAUNA

(30) Priorität: 02.02.2021 AT 600332021
(43) Veröffentlichungstag der Anmeldung: 13.12.2023
(73) Patentinhaber: KXR Kälte + Anlagentechnik GmbH, 8324 Kirchberg an der Raab (AT)
(72) Erfinder: KRAXNER, David, 8324 Kirchberg an der Raab (AT); SCHÄFFER, Markus, 8421 Schwarzautal (AT); SUPPAN, Michael, 8421 Schwarzautal (AT)
(74) Vertreter: Wirnsberger & Lerchbaum Patentanwälte OG
(86) Internationale Anmeldenummer: PCT/AT2021/060351
(87) Internationale Veröffentlichungsnummer: WO 2022/165543

(56) Entgegenhaltungen:
- AT-A1- 524 726
- BE-A- 341 687
- BE-A- 412 050
- DE-A1- 19 917 974
- DE-U1- 202019 104 355
- GB-A- 421 145
- GB-A- 468 569
- JP-A- 2006 029 713
- JP-U- H0 651 769
- US-A- 1 734 127
- US-A- 1 794 030
- US-A- 2 882 701
- US-A- 4 353 223

## Beschreibung

Die Erfindung betrifft eine Cryosauna gemäß dem Oberbegriff des Anspruchs 1.

Des Weiteren betrifft die Erfindung ein Verfahren zur Herstellung einer Temperaturhomogenität in einer Cryosauna.

Aus dem Stand der Technik sind Kältekammern bekannt, welche oftmals auch als Tieftemperaturkammern, Cryokammern bzw. Kryokammern oder Cryosauna bezeichnet werden. Bei derartigen Kältekammern kommen in der Regel Ventilatoren zum Einsatz, um die Luft im Inneren der Kammer umzuwälzen. Bei diesem Prinzip spricht man in Fachkreisen auch von einer Zwangsumwälzung. Die Umwälzung dient dem Zweck, das Leistungsvermögen eines Verdampfers oder Wärmetauschers bestmöglich nutzen und die Kältekammer effizient abkühlen zu können. Dabei führt die Umwälzung zu einem Ausgleich der Lufttemperatur zwischen den kältesten Bereichen der Kältekammer, welche sich zumeist nahe dem Boden bzw. dem Verdampferm oder Wärmetauscher befinden, und den wärmsten Bereichen der Kältekammer, welche oftmals nahe der Decke bzw. den am weitesten vom Verdampfer oder Wärmetauscher entfernten Bereichen der Kältekammer befinden.

Ein Einsatz von Ventilatoren zur Umwälzung führt zu Luftbewegungen und Luftströmungsgeschwindigkeiten innerhalb der Kältekammer, die von Nutzern, welche sich in der Regel zu medizinischen Behandlungszwecken in die Kältekammer begeben, als störend empfunden werden. Zudem führen tiefe Temperaturen sowie Eis- und Schneebildung zu vermehrten Ausfällen, einer Kurzlebigkeit oder Störanfälligkeit der Ventilatoren, welche zumeist deutlich außerhalb der Spezifikationsgrenzen betrieben werden.

Die JP H06 51769 U offenbart ein Warenhaus mit einem Innenraum, wobei im Innenraum Luft zur Kühlung des Innenraums in einem Kreislauf umgewälzt wird.

Die DE 10 2019 104 355 U1 offenbart eine Kältesauna für die Sport- und Schmerztherapie. Die Kältesauna weist einen äußeren Behandlungsraum und einen inneren Behandlungsraum auf, wobei eine Temperatur im äußeren Behandlungsraum höher ist als im inneren Behandlungsraum. Der äußere Behandlungsraum fungiert als Kälteschild für den inneren Behandlungsraum. Zur Einstellung einer Temperatur im inneren Behandlungsraum wird Luft im Umluftbetrieb zirkuliert, wofür entsprechende Zuluft- und Abluftkanäle vorgesehen sind.

Aufgabe der Erfindung ist es, eine Cryosauna der eingangs genannten Art bereitzustellen, welche die Nachteile des Standes der Technik vermeidet und gleichzeitig eine hohe Temperaturhomogenität erzielt.

Ein weiteres Ziel der Erfindung ist es, ein Verfahren der eingangs genannten Art entsprechend weiterzubilden.

Diese Aufgabe wird erfindungsgemäß mit einer Cryosauna nach Anspruch 1 gelöst.

Ein statischer Verdampfer ist ein Verdampfer, mit dem die Kühlleistung ohne Zwangsumwälzung erreicht wird. Dementsprechend werden statische Verdampfer auch als stille Verdampfer bezeichnet.

Im Rahmen der Erfindung ist es bevorzugt, dass bei bzw. in der Kältekammer nur ein oder mehrere statische Verdampfer eingesetzt werden. Es erfolgt dann keine Zwangsumwälzung.

Zudem wird zumindest ein Leitblech zur Optimierung der Verteilung der thermischen Energie genutzt, woraus vor allem in einem oberen Bereich der Kältekammer eine Verbesserung der Temperaturschichtung, insbesondere der Temperaturhomogenität, resultiert. Hierbei wird ein Leitblech vertikal vor dem Verdampfer bzw. Wärmetauscher angebracht und ein Leitblech horizontal nahe der Decke der Kältekammer. Zum Erreichen der Wirkung ist es jedoch unerheblich, ob es sich um zwei trennbare Leitbleche oder ein gesamtes Leitblech handelt.

Darüber hinaus ist das Erreichen der Wirkung nicht an die Verwendung beider Leitbleche gebunden. Eine Verbesserung gegenüber dem Stand der Technik kann bereits durch die Verwendung nur eines der beiden Leitbleche, das vertikale und/oder deckennah bzw. deckennah und/oder vertikal angeordnete Leitblech, erzielt werden. Eine Trennung der Leitbleche erfolgt lediglich wegen der Einbringung in die Kältekammer.

Ein Material bzw. eine Oberflächenbeschaffenheit der Leitbleche wirkt sich auf eine Effektivität der Leitbleche aus. Dabei verbessert sich mit einer höheren Wärmeleitfähigkeit des Materials auch die Temperaturhomogenität. Eingesetzt werden Edelstahlbleche und/oder Aluminiumbleche.

Zudem wird die Temperaturhomogenität durch eine Ausbildung der Leitbleche mit einer möglichst großen Oberfläche positiv beeinflusst. Außenabmessungen der Leitbleche werden durch die Größe der Kältekammer beschränkt.

Darüber hinaus wird die Temperaturhomogenität positiv beeinflusst, indem die Oberfläche der Leitbleche bei identen Außenabmessungen erhöht wird. Dies wird mit speziellen Ausformungen, insbesondere Kantungen, realisiert.

Ein weiterer positiver Effekt auf die Temperaturhomogenität wird erzielt, indem in das Leitblech ein Kältekreis, insbesondere ein Kälterohr, eingearbeitet wird.

Außerdem wird die Temperaturhomogenität positiv beeinflusst, indem Lamellen bzw. Rippen am Kälterohr in dem oder den Leitblechen eingearbeitet werden.

Durch eine erfindungsgemäße Kältekammer wird die Luftbewegung ausschließlich durch natürliche Konvektion erreicht und somit auf ein physikalisches Minimum reduziert. Gleichermaßen stellt sich die niedrigste Luftströmungsgeschwindigkeit ein. Dies bietet vor allem Vorteile für Personen, welche sich beispielsweise zu medizinischen Behandlungszwecken in die Kältekammer begeben.

Zudem wird durch die erfindungsgemäße Kältekammer die "Kälteverteilung" im Raum und damit die Temperaturhomogenität gegenüber einem Aufbau mit statischem Verdampfer ohne Leitbleche optimiert.

Darüber hinaus wird mit einer erfindungsgemäßen Kältekammer, im Vergleich mit anderen auf dem Markt bestehenden Produkten, hinsichtlich der Raumhöhe eine niedrigste und damit optimierteste Variante erzielt.

Außerdem werden mit einer erfindungsgemäßen Kältekammer Anlagenstörungen reduziert. Ventilatoren haben eine hohe Ausfallrate im Tieftemperaturbereich. Tiefe Temperaturen und Eis- bzw. Schneebildung führen zu vermehrten Ausfällen der Ventilatoren. Zudem geht mit diesem Einsatzbereich, welcher meist deutlich außerhalb der Spezifikationsgrenzen der Ventilatoren liegt, eine Kurzlebigkeit und Störanfälligkeit der Ventilatoren einher.

Das verfahrensmäßige Ziel wird erreicht, wenn bei einem Verfahren der eingangs genannten Art die Temperaturhomogenität über ein vertikal vor einem statischen Verdampfer bzw.

Wärmetauscher angeordnetes Leitblech und ein nahe einer Decke der Kältekammer angeordnetes zweites Leitblech verbessert wird. Hierbei kommen bevorzugt wiederum nur ein oder mehrere statische Verdampfer zum Einsatz.

In den Zeichnungen ist gezeigt:
Fig. 1 schematische Darstellung einer Kältekammer samt Verdampfer und Leitblechen;
Fig. 2 Schnittansicht der Kältekammer gemäß Fig. 1;
Fig. 3 Schnittansicht der Kältekammer samt Verdampfer und Leitblechen mit integrierter Kälteleitung und Übertragungslamellen bzw. Übertragungsrippen;
Fig. 4 schematische Darstellungen dreier Ausführungsvarianten eines Leitbleches.

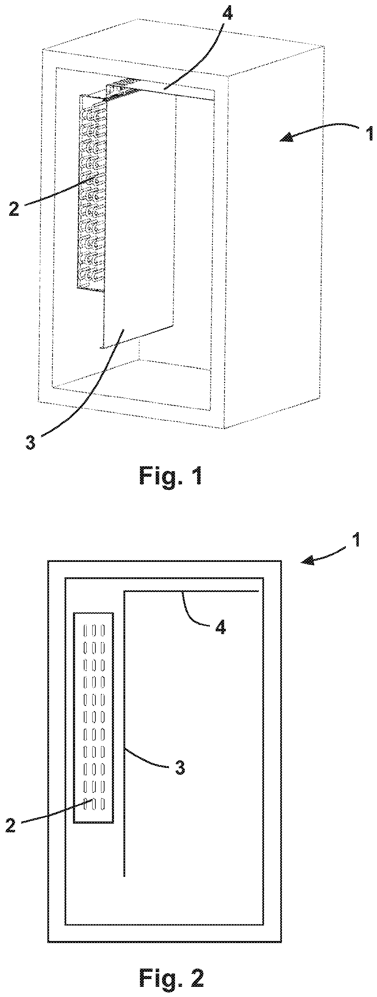

In Fig. 1 ist eine Kältekammer 1 mit an einer Seite verbautem statischen Verdampfer 2 gezeigt. Zudem sind ein vor dem statischen Verdampfer montiertes vertikales Leitblech 3 und ein an einer Decke montiertes horizontales Leitblech 4 ersichtlich.

In der Fig. 2 ist eine Schnittansicht der Kältekammer 1 gemäß Fig. 1 dargestellt. Dabei ist die Kältekammer 1 mit an der Seite verbautem statischen Verdampfer 2 und davor montiertem vertikalen Leitblech 3 sowie an der Decke montiertem horizontalen Leitblech 4 gezeigt.

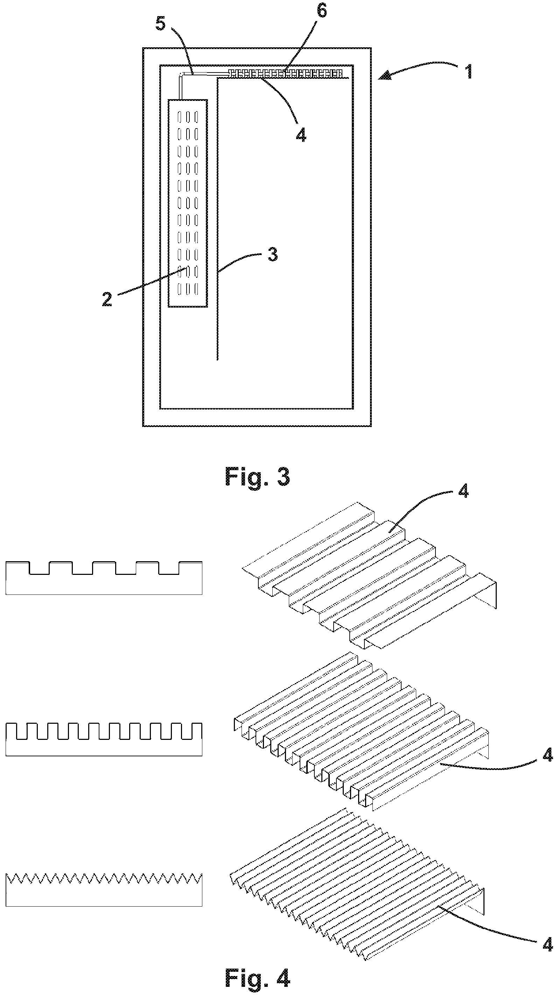

In Fig. 3 ist die Schnittansicht einer Kältekammer 1 samt Verdampfer 2 und Leitblechen 3, 4 mit integrierter Kälteleitung 5 und Übertragungslamellen 6 bzw. Übertragungsrippen dargestellt. Hierbei ist die Kältekammer 1 mit an der Seite verbautem statischen Verdampfer 2 und davor montiertem vertikalen Leitblech 3 gezeigt. Zudem weist das an der Decke montierte horizontale Leitblech 4 eine integrierte Kälteleitung 5 und Übertragungslamellen 6 auf. Die Übertragungslamellen 6 dienen einer Anbindung der Kälteleitung 5 an das Leitblech 4 und damit einer zusätzlichen Kühlung des Leitbleches 4 durch ein Kühlmedium in der Kälteleitung 5.

In der Fig. 4 ist eine schematische Darstellung dreier Ausführungsvarianten eines Leitbleches 4 gezeigt. Dabei dienen unterschiedliche Ausformungen der Leitbleche 3, 4 einer Erhöhung einer Leitblechoberfläche bei identen Außenabmessungen, insbesondere Länge und Breite, der Leitbleche 3, 4.

## Patentansprüche

1. Cryosauna, welche zum Kühlen einen Verdampfer (2) und/oder Wärmetauscher und zumindest ein Leitblech (3, 4) aufweist, wobei der Verdampfer (2) als statischer Verdampfer ausgebildet ist, **dadurch gekennzeichnet, dass** zur Verbesserung einer Temperaturhomogenität ein erstes Leitblech (3) vertikal vor dem Verdampfer (2) bzw. Wärmetauscher und ein zweites Leitblech (4) horizontal nahe einer Decke der Cryosauna angeordnet ist.

2. Cryosauna nach Anspruch 1, **dadurch gekennzeichnet, dass** der Verdampfer (2) und/oder Wärmetauscher an einer Wand und/oder einer Decke der Cryosauna befestigt ist.

3. Cryosauna nach Anspruch 1, **dadurch gekennzeichnet, dass** das Leitblech (3, 4) aus einem Material mit hoher thermischer Leitfähigkeit, insbesondere Edelstahl und/oder Aluminium, gebildet ist.

4. Cryosauna nach Anspruch 1, **dadurch gekennzeichnet, dass** die Leitbleche (3, 4) zur Erhöhung einer Oberfläche mit mehreren Kantungen versehen sind.

5. Cryosauna nach Anspruch 1, **dadurch gekennzeichnet, dass** die Cryosauna zur Kühlung mehrere Verdampfer (2) und zur Verbesserung der Temperaturhomogenität mehrere Leitbleche (3, 4) aufweist.

6. Cryosauna nach Anspruch 1, **dadurch gekennzeichnet, dass** das zweite Leitblech (4) eine integrierte Kälteleitung (5) aufweist.

7. Cryosauna nach Anspruch 1, **dadurch gekennzeichnet, dass** eine Kälteleitung vorgesehen ist, welche über Lamellen (6) oder Rippen mit dem zweiten Leitblech (4) verbunden ist.

8. Cryosauna nach Anspruch 7, **dadurch gekennzeichnet, dass** das zweite Leitblech (4) mit integrierter Kälteleitung (5) und Übertragungslamellen (6) und/oder Übertragungsrippen ausgeführt ist.

9. Cryosauna nach Anspruch 8, **dadurch gekennzeichnet, dass** die integrierte Kälteleitung (5) über Lamellen (6) und/oder Rippen an dem zweiten Leitblech (4) angeordnet ist.

10. Verfahren zur Herstellung einer Temperaturhomogenität in einer Cryosauna gemäß einem der Ansprüche 1 bis 9, **dadurch gekennzeichnet, dass** die Temperaturhomogenität über ein vertikal vor einem Verdampfer (2) bzw. Wärmetauscher angeordnetes erstes Leitblech (3) und ein nahe einer Decke der Kältekammer (1) angeordnetes zweites Leitblech (4) verbessert wird.

11. Verfahren nach Anspruch 10, **dadurch gekennzeichnet, dass** zur Herstellung der Temperaturhomogenität keine Zwangsumwälzung, insbesondere nicht durch einen Ventilator, erfolgt.

## Claims

1. A cryosauna, which comprises an evaporator (2) and/or a heat exchanger and at least one baffle plate (3, 4) for cooling, wherein the evaporator (2) is designed as a static evaporator, **characterised in that** a first baffle plate (3) is arranged vertically in front of the evaporator (2) or heat exchanger and a second baffle plate (4) is arranged horizontally close to a cover of the cryosauna for the purpose of improving temperature homogeneity.

2. The cryosauana according to claim 1, **characterised in that** the evaporator (2) and/or heat exchanger is fastened to a wall and/or a cover of the cryosauana.

3. The cryosauana according to claim 1, **characterised in that** the baffle plate (3, 4) is made of a material with high thermal conductivity, in particular stainless steel and/or aluminium.

4. The cryosauana according to claim 1, **characterised in that** the baffle plates (3, 4) are provided with a plurality of folds in order to increase a surface area.

5. The cryosauana according to claim 1, **characterised in that** the cryosauana comprises a plurality of evaporators (2) for cooling and a plurality of baffle plates (3, 4) to improve the temperature homogeneity.

6. The cryosauana according to claim 1, **characterised in that** the second baffle plate (4) comprises an integrated refrigerant line (5).

7. The cryosauana according to claim 1, **characterised in that** a refrigerant line is provided which is connected via fins (6) or ribs to the second baffle plate (4).

8. The cryosauana according to claim 7, **characterised in that** the second baffle plate (4) is designed with an integrated refrigerant line (5) and transmission fins (6) and/or transmission ribs.

9. The cryosauana according to claim 8, **characterised in that** the integrated refrigerant line (5) is arranged via fins (6) and/or ribs on the second baffle plate (4) .

10. A method for creating a temperature homogeneity in a cryosauana according to any one of claims 1 to 9, **characterised in that** the temperature homogeneity is improved by a first baffle plate (3) arranged vertically in front of an evaporator (2) and/or heat exchanger and a second baffle plate (4) arranged close to a cover of the cold chamber (1).

11. The method according to claim 10, **characterised in that** no forced circulation, in particular by means of a ventilator, takes place in order to create the temperature homogeneity.

## Revendications

1. Cryosauna, lequel comporte pour refroidir un évaporateur (2) et/ou un échangeur de chaleur et au moins une tôle de guidage (3, 4), sachant que l'évaporateur (2) est constitué sous la forme d'un évaporateur statique, **caractérisé en ce qu'**une première tôle de guidage (3) est disposée verticalement avant l'évaporateur (2) ou l'échangeur de chaleur et une deuxième tôle de guidage (4) est disposée horizontalement près d'un plafond du cryosauna pour améliorer une homogénéité de température.

2. Cryosauna selon la revendication 1, **caractérisé en ce que** l'évaporateur (2) et/ou l'échangeur de chaleur est fixé sur une paroi et/ou un plafond du cryosauna.

3. Cryosauna selon la revendication 1, **caractérisé en ce que** la tôle de guidage (3, 4) est formée dans un matériau avec une thermoconductivité élevée, en particulier en acier spécial et/ou en aluminium.

4. Cryosauna selon la revendication 1, **caractérisé en ce que** les tôles de guidage s (3, 4) sont dotées de plusieurs arêtes pour augmenter une surface.

5. Cryosauna selon la revendication 1, **caractérisé en ce que** le cryosauna comporte plusieurs évaporateurs (2) pour le refroidissement et plusieurs tôles de guidage s (3, 4) pour améliorer l'homogénéité de température.

6. Cryosauna selon la revendication 1, **caractérisé en ce que** la deuxième tôle de guidage (4) comporte une conduite de froid intégrée (5).

7. Cryosauna selon la revendication 1, **caractérisé en ce qu'**une conduite de froid est prévue, laquelle est reliée par des lamelles (6) ou des nervures à la deuxième tôle de guidage (4).

8. Cryosauna selon la revendication 7, **caractérisé en ce que** la deuxième tôle de guidage (4) est exécutée avec une conduite de froid intégrée (5) et des lamelles de transmission (6) et/ou des nervures de transmission.

9. Cryosauna selon la revendication 8, **caractérisé en ce que** la conduite de froid (5) intégrée est disposée au-dessus des lamelles (6) et/ou des nervures sur la deuxième tôle de guidage (4).

10. Procédé de réalisation d'une homogénéité de température dans un cryosauna selon l'une quelconque des revendications 1 à 9, **caractérisé en ce que** l'homogénéité de température est améliorée par une première tôle de guidage (3) disposée verticalement avant un évaporateur (2) ou échangeur de chaleur et une deuxième tôle de guidage (4) disposée près d'un plafond de la chambre froide (1).

11. Procédé selon la revendication 10, **caractérisé en ce qu'**aucune recirculation forcée ne se produit, en particulier pas par un ventilateur, pour réaliser l'homogénéité de température.
